# EUROPEAN PATENT SPECIFICATION

(11) **EP 0 561 913 B1**
(45) Date of publication and mention of the grant of the patent: **01.04.1998**
(21) Application number: 92900842.3
(22) Date of filing: 13.11.1991
(51) Int. Cl.: A61K 31/435, A61K 31/44

(54) **USE OF PYRIDINE DERIVATIVES IN THE TREATMENT OF OCULAR HYPERTENSION**
VERWENDUNG VON PYRIDINE DERIVATE ZUR BEHANDLUNG DER OKULÄREN HYPERTOMIE
UTILISATION DE DERIVES DE PYRIDINE POUR TRAITER L'HYPERTENSION OCULAIRE

(30) Priority: 29.11.1990 US 620841
(43) Date of publication of application: 29.09.1993
(73) Proprietor: ALLERGAN, INC., Irvine, California 92623-9534 (US)
(72) Inventor: WOLDE MUSSIE, Elizabeth, Costa Mesa, CA 92626 (US); CHEN, June, San Juan Capistrano, CA 92675 (US); RUIZ, Guadalupe, Corona, CA 91720 (US)
(74) Representative: Griffin, Kenneth David
(86) International application number: US9108484
(87) International publication number: WO9209280

(56) References cited:
- EP-A- 0 259 621
- EP-A- 0 296 721
- EP-A- 0 307 141
- EP-A- 0 316 718
- EP-A- 0 384 288
- EP-A- 0 459 568
- Ph. DOROSZ: "Guide pratique des Médicaments", 7th edition, 1977, Maloine S.A., (Paris, FR), pages 816-817, see "Collyres antiglaucomateux: Pilocarpine, Acéclidine"
- Journal of Medicinal Chem., vol. 33, no. 10, October 1990, L.J. STREET et al.: "Synthesis and biological activity of 1,2,4-oxadiazole derivatives: Highly potent and afficacious agonists for cortical muscarinic receptors", pages 2690- 2697, see whole document
- Journal of Medicinal Chem., vol. 33, no. 7, July 1990, A.M. MacLEOD et al.: "Synthesis and muscarinic activities of 1,2,4-thiadiazoles", pages 2052-2059, see whole document

## Description

### Field of the Invention

The present invention relates to the use of certain pyridine derivatives in the treatment of ocular hypertension.

### Background of the Invention

Ocular hypotensive agents are useful in the treatment of a number of various ocular hypertensive conditions, such as post-surgical and post-laser trabeculectomy ocular hypertensive episodes, glaucoma, and as presurgical adjuncts.

Glaucoma is a disease of the eye characterized by increased intraocular pressure. On the basis of its etiology, glaucoma has been classified as primary or secondary. For example, primary glaucoma in adults may be either open-angle or acute or chronic angle-closure. Secondary glaucoma results from pre-existing ocular diseases such as uveitis, intraocular tumor or an enlarged cataract.

Primary open angle glaucoma is a disease of the optic nerve which is associated with an increase in intraocular pressure (IOP). This increase is due to increased resistance to aqueous humor outflow at the trabecular meshwork [Johnson, D.H. and Brubaker, R.F.: Glaucoma: An overview. Mayo Clin. Proc., G1:59-67 (1986)].

In chronic open-angle glaucoma, the anterior chamber and its anatomic structures appear normal, but drainage of the aqueous humor is impeded. In acute or chronic angle-closure glaucoma, the anterior chamber is shallow, the filtration angle is narrowed, and the iris may obstruct the trabecular meshwork at the entrance of the canal of Schlemm. Dilation of the pupil may push the root of the iris forward against the angle, and may produce pupillary block and thus precipitate an acute attack. Eyes with narrow anterior chamber angles are predisposed to acute angle-closure glaucoma attacks of various degrees of severity.

Secondary glaucoma is caused by any interference with the flow of aqueous humor from the posterior chamber into the anterior chamber and subsequently, into the canal of Schlemm. Inflammatory disease of the anterior segment may prevent aqueous escape by causing complete posterior synechia in iris bombe, and may plug the drainage channel with exudates. Other common causes are intraocular tumors, enlarged cataracts, central retinal vein occlusion, trauma to the eye, operative procedures and intraocular hemorrhage.

Considering all types together, glaucoma occurs in about 2% of all persons over the age of 40 and may be asymptotic for years before progressing to rapid loss of vision.

One drug that has been used for the treatment of glaucoma for over a hundred years is pilocarpine [(3S,4R)-3-ethyl-4-{(1-methyl-1H-imidazol-5-yl)methyl}-3,4-dihydro-2(3H)-furanone]. Pilocarpine is a muscarinic agonist that reduces resistance to aqueous humor outflow by causing contraction of the ciliary muscle, thus pulling the trabecular mechwork apart. [See, e.g. Barany, E.H., Invest. Ophthalmol. 1, 712-727 (1962); and Kaufman, P.L. and Barany, E.H., Invest. Ophthalmol. 15, 793-807 (1976).] Although pilocarpine is a safe and effective drug, it has undesired side effects such as miosis (reduction of pupil size) due to contraction of the iris sphincter muscle. It also has short duration of action, and therefore, has to be administered four-times a day which creates a problem with patient compliance. These undesired properties have limited the wide application of pilocarpine and other muscarinics, and in the past decade other compounds with different mechanism of action such as inhibition of aqueous humor formation, have been the drugs of choice.

A well known compound acting via reduction of the production of the aqueous humor is epinephrine (also called adrenaline), by its most commonly used chemical name 4-[1-hydroxy-2-(methylamino)ethyl]-1,2-benzenediol. Its 1-form has adrenergic properties, and together with its dipivalate ester derivative, propine, is a clinically useful anti-glaucoma agent. However, these compounds also cause mydriasis, an excessive dilation of the pupil of the patient's eye upon administration.

Certain eicosanoids and their derivatives have been reported to possess ocular hypotensive activity, and have been recommended for use in glaucoma management. Eicosanoids and derivatives include numerous biologically important compounds such as prostaglandins and their derivatives.

Certain compounds, structurally similar to the muscarinic agonist arecoline (1,2,5,6-tetrahydro-1-methyl-3-pyridinecarboxylic acid methyl ester) were disclosed in the European Patent Applications Publication Nos. 259,621, 296,721, 316,718 and 384,288 as suitable for the treatment of cognitive disorders such as Alzheimer's disease.

### Summary of the Invention

In search of muscarinic agonists, we have identified a group of compounds which effectively reduce ocular pressure but are substantially devoid of the undesired side effects of pilocarpine and its analogs. These compounds are encompassed by the formula (I) 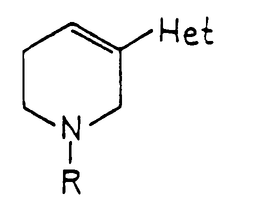 where R is hydrogen or an acyclic hydrocarbon group of about 1 to 6 carbon atoms, and Het is a five membered heterocyclic ring containing two hetero-atoms selected from N, O and S, unsubstituted or substituted with an acyclic hydrocarbon group of up to about 10 carbon atoms, or an alicyclic hydrocarbon group of about 3 to 6 carbon atoms, or with an -OR₁ group in which R₁ is an acyclic hydrocarbon group of 1 to about 6 carbon atoms. These and structurally related compounds were disclosed in EP 296,721 US 4,866,077), EP 316,718 and EP 259,621 as structural analogs of arecoline, a cholinergic agonist. Since the disclosed compounds were described as having potent acetylcholine activity indicating utility in the treatment of diseases caused by reduced function of acetylcholine in the brain, in particular, Alzheimer's disease, their ocular hypotensive activity is highly unexpected.

In one aspect, the present invention relates to the use of such compounds for the preparation of a medicament for the treatment of ocular hypertension which comprises applying to the eye an amount sufficient to treat ocular hypertension of a compound of the above formula (I), wherein the substituents are as hereinabove defined, or a pharmaceutically acceptable acid addition salt thereof.

In another aspect, the invention relates to an ophthalmic solution for the treatment of ocular hypertension, comprising an amount sufficient to treat ocular hypertension of a compound of formula (I) as hereinabove defined, or a pharmaceutically acceptable acid addition salt thereof, in admixture with a non-toxic, ophthalmically acceptable liquid vehicle, packaged in a container suitable for metered application.

In a further aspect, the invention relates to a pharmaceutical product, comprising
a container adapted to dispense its contents in metered form; and
an ophthalmic solution therein, as hereinabove defined.

### Brief Description of the Figures

Figure 1 illustrates the effect of pilocarpine on intraocular pressure (IOP) in rabbits. Pilocarpine (10 µg/20 µl) (□) or saline (o) was injected intracamerally and IOP was measured at indicated times.

Figure 2 shows the effect of pyridine-1,2,3,6-tetrahydro-5-(5-isoxazolyl)-1-methyl-ethanedioate (test compound) on IOP in rabbits. The test compound (10 µg/20 µl) (•) or saline (o) was injected intracamerally and IOP was measured at indicated times.

Figure 3 shows the effect of pilocarpine on IOP in owl monkeys. Pilocarpine (□) (200 µg/10 µl) was applied topically. Control animals received saline (o).

Figure 4 illustrates the effect of the test compound on IOP in owl monkeys. The test compound (200 µg/10 µl) (•) was applied topically. Control animals received saline (o).

Figure 5 shows the effect of atropine on the test compound-induced decrease in IOP in rabbits. Atropine (5 µg/20 µl) was injected with the test compound (10 µg/20 µl) (□) and IOP was measured. Values are compared with the test compound alone (•) or saline (o).

Figure 6 shows the effect of atropine on the test compound-induced reduction in IOP in owl monkeys. Atropine was applied topically (5 µg/10 µl) (□). Values are compared with the test compound alone (200 µg/10 µl) (•) or saline (o).

Figure 7 illustrates the decrease in pupil size induced by intracamerally applied pilocarpine (10 µg/20 µl) (□). Control animals received saline (o).

Figure 8 shows the decrease in pupil size in rabbits induced by intracamerally applied test compound (10 µg/20 µl) (•). Control animals received saline (o).

Figure 9 shows the effect of pilocarpine on pupil size in owl monkeys. Pilocarpine (200 µg/µl) (□) was applied topically. Control animals received saline (o).

Figure 10 illustrates the effect of the test compound on pupil size in owl monkeys. The test compound (200 µg/10 µl) (•) was applied topically. Control animals received saline (o).

Figure 11 shows the effect of atropine on the test compound-induced miosis in rabbits. Pupil diameter was measured at indicated times following intracameral injection of 10 µg/20 µl test compound (□) or test compound and 5 µg atropine (•). Saline (o) was injected into control animals.

Figure 12 shows the effect of atropine on test compound-induced miosis in owl monkeys. Atropine (□) (5 µg/10 µl) was applied topically. Values are compared to the test compound alone (•) or saline (o).

Figure 13 shows the effect of the test compound on contraction of cat ciliary (□) or iris sphincter (•) smooth muscle.

Figure 14 illustrates the effect of atropine (open symbols) on test compound-induced contraction of cat ciliary or iris sphincter smooth muscle. Tissues were incubated with 10nM atropine as described in the Examples. Test compound-induced contraction is shown by closed symbols.

### Detailed Description of the Invention

The present invention is based on the unexpected finding that certain analogs of the muscarinic agonist arecoline cause a reduction in intraocular pressure with minimal effect on pupil size, and have a long duration of action.

The compounds used in accordance with the present invention have the following structure 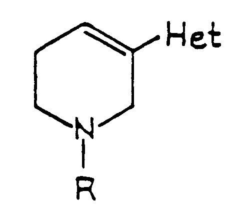 wherein R is hydrogen or an acyclic hydrocarbon group of 1 to about 6 carbon atoms, and Het is a five membered heterocyclic ring containing two hetero-atoms selected from N, O and S, unsubstituted or substituted with an acyclic hydrocarbon group of up to about 10 carbon atoms, or an alicyclic hydrocarbon group of about 3 to 6 carbon atoms, or with an -OR₁ group in which R₁ is an acyclic hydrocarbon group of 1 to about 6 carbon atoms.

Het as a five membered heterocyclic group may include one or two double bonds, and preferably is selected from

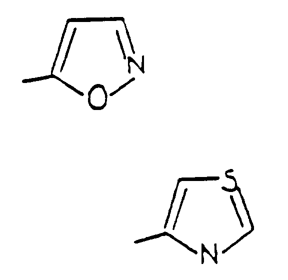

In the above substituent definitions, the term "acyclic hydrocarbon group" can be saturated or unsaturated, and is used to refer to a straight or branched chained saturated or unsaturated hydrocarbon groups of appropriate lengths, including straight and branched chained alkyl, alkenyl and alkinyl groups. Within this definition, preferred are the alkyl groups, preferably having 1 to about 6 carbon atoms, such as methyl, ethyl, n-and i-propyl, n- and t-butyl, n-and i-pentyl, n-and i-hexyl group, etc. R preferably is an alkyl group having 1 to 4 carbon atoms, more preferably 1 or 2 carbon atoms. In the most preferred compounds of formula (I) R is a methyl group. In the definition of R₁, the preferred acyclic hydrocarbon groups are those having up to 4 carbon atoms.

The term "alicyclic hydrocarbon group" is used to refer to a saturated or unsaturated cyclic hydrocarbon group having from 3 to about 6 carbon atoms, and includes cyclopropyl, cyclopentyl, cyclopentenyl, cyclohexyl and cyclohexenyl groups, etc.

In a particular preferred group of the compounds according to the present invention the Het heterocyclic ring is an isoxazole or thiazole ring.

Particularly preferred are the following compounds:
pyridine-1,2,3,6-tetrahydro-5-(5-isoxazolyl)-1-methyl-ethanedioate;
pyridine-1,2,3,6-tetrahydro-5-(2-methyl-4-thiazolyl)-1-methyl-ethanedioate;
   and the pharmaceutically acceptable acid addition salts of these compounds.

The pharmaceutically acceptable acid addition salts of the compounds of formula (I) contain an anion which is not toxic in usual therapeutic doses. Preferred acid addition salts include hydrochlorides, hydrobromides, sulphates, acetates, phosphates, nitrates, methanesulphonates, ethanesulphonates, lactates, citrates, tartarates, etc. Further acids suitable for forming pharmaceutically acceptable acid addition salts are, for example, fumaric, benzoic, succinic, palmitic, benzenesulphonic, etc. acids.

The compounds of the formula (I) are either specifically disclosed in one or more of the above-mentioned European Patent Applications, or can be prepared from known compounds by methods known in the art of organic chemistry.

The disclosed compounds exhibit valuable pharmaceutical properties. More particularly, these compounds are potent anti-glaucoma agents that are essentially devoid of the undesirable side-effects of the known muscarinic agonist anti-glaucoma agents currently used in clinical practice, such as pilocarpine. Moreover, these compounds have a significantly longer duration of action.

Pharmaceutical compositions may be prepared by combining a therapeutically efficient amount of at least one compound according to the present invention, or a pharmaceutically acceptable acid addition salt thereof, as an active ingredient, with conventional pharmaceutical excipients. Preferably, at least one of the active ingredients is a compound of formula (I), wherein the substituents are as hereinabove defined. The therapeutically efficient amount typically is between about 0.1 and about 5 % (w/v) in liquid formulations.

For ophthalmic application, preferably solutions are prepared using a physiological saline solution as a major vehicle. The pH of such ophthalmic solutions should preferably be maintained between 6.5 and 7.2 with an appropriate buffer system. The formulations may also contain conventional, pharmaceutically acceptable preservatives and stabilizers.

Preferred preservatives that may be used in the pharmaceutical compositions of the present invention include, but are not limited to, benzalkonium chloride, chlorobutanol, thimerosal, phenylmercuric acetate and phenylmercuric nitrate. Likewise, various preferred vehicles may be used in the ophthalmic preparations of the present invention. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In a similar vein, an ophthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edetate disodium, although other chelating agents may also be used in place or in conjunction with it.

The ingredients are usually used in the following amounts:

| Ingredient | Amount (% w/v) |
|---|---|
| active ingredient | about 0.1-5 |
| preservative | 0-0.10 |
| vehicle | 0-40 |
| tonicity adjustor | 0-10 |
| buffer | 0.01-10 |
| pH adjustor | q.s. pH 4.5-7.5 |
| antioxidant | as needed |
| purified water | as needed to make 100% |

The pharmacological activity of the compounds according to the present invention is further illustrated by the following non-limiting Examples.

### Example 1

In vivo study of intraocular pressure reducing activity.

### Methods

Pyridine-1,2,3,6-tetrahydro-5-(5-isoxazolyl)-1-methyl-ethanedioate ("test compound") or pilocarpine were applied either topically into the eyes of owl monkeys (200 µg in 10 µl) or intracamerally into the eye of rabbits (10 µg in 20 µl). Control animals were treated with a saline vehicle. Pupil diameter and IOP were measured for 0-6 hours. IOP was measured using pneumatometer (Digilab/ Biorad) and pupil diameter was measured using Optistick ruler (Allergan). Animals used in these studies had normal intraocular pressure.

Muscarinic activity was determined by treatment with atropine (muscarinic antagonist). In rabbits 5 µg atropine was coinjected with the test compounds and in owl monkey 5 µg atropine (10 µl) was applied 30 minutes before treatment with the test compound.

Intraocular pressure (mmHg) or pupil diameter (mm) were expressed as changes from time 0.

### Results and Discussion

Intraocular pressure. Muscarinic agonists such as pilocarpine cause little or no decrease in IOP in normotensive rabbits or humans [Miichi, H. and Nagataki, S., J. ophthalmol. 26, 4250436 (1982); Naveh-Floman et al., Ophthalmic Res. 18, 34-37 (1986)]. The owl monkey is one of the few species that responds to pilocarpine [Lamble, et al., Invest. Ophthalmol. 15, 848-851 (1976)].

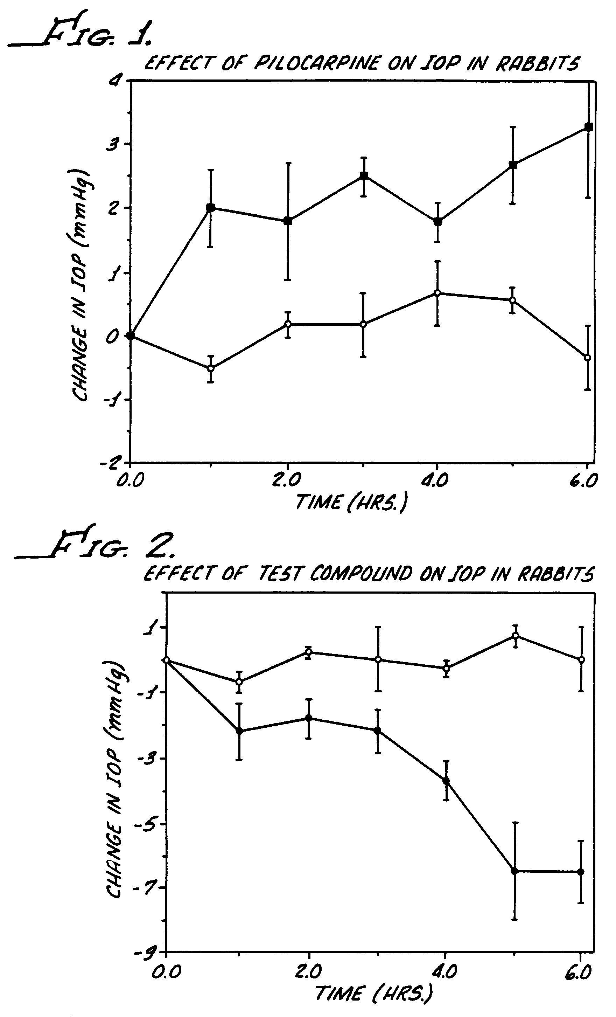

The results presented in Figure 1 confirm this observation on pilocarpine's effect in rabbits. Pilocarpine (10 µg/20 µl) injected intracamerally showed no hypotensive effect. In fact, as shown in Figure 1, there was a slight hypertensive action.

This study also shows that the test compound differs from pilocarpine with respect to its effect on IOP in rabbits. When applied intracamerally, the test compound caused decrease in IOP of 6 mmHg 5 hours after administration (Figure 2). This activity appeared to last longer than 6 hours which was the end of the experimental period. This showed that not only did the test compound cause a decrease in IOP, but also had a longer duration of action.

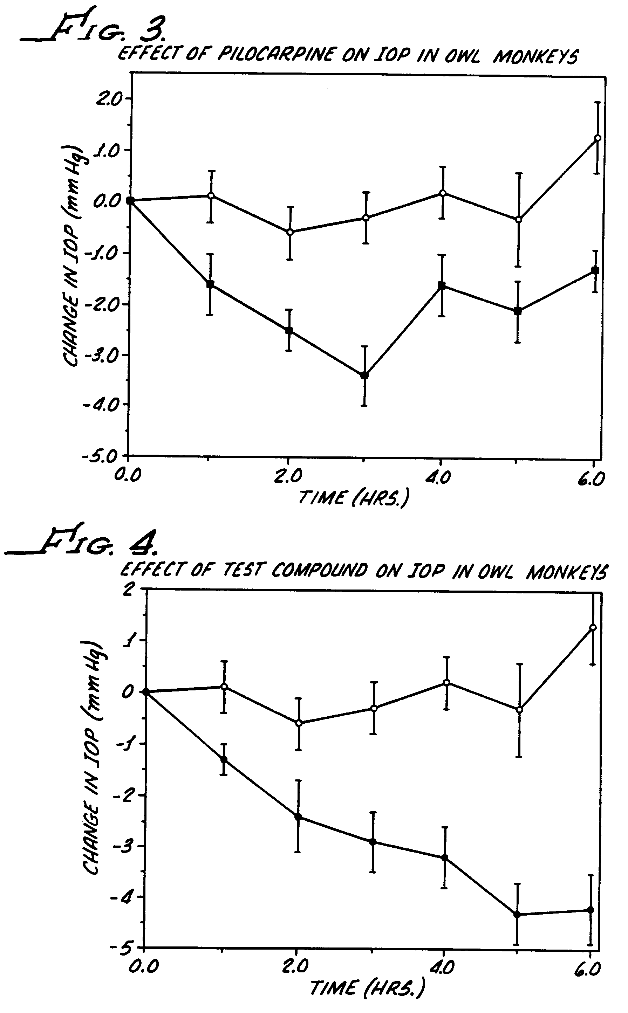

As mentioned above, the owl monkey is one of the few normotensive animals that respond to pilocarpine. As expected, pilocarpine caused a decrease in IOP of about 3 mmHg at 3 hours and the effect decreased thereafter (Figure 3).

Application of the test compound into the eyes of owl monkeys also caused reduction in IOP with maximum decrease of 4 mmHg after 5 and 6 hours (Figure 4). Here again, as in the case of rabbits, the effect lasted longer than the experimental period of 6 hours, indicating that the test compound has a longer duration than that of pilocarpine in the owl monkey.

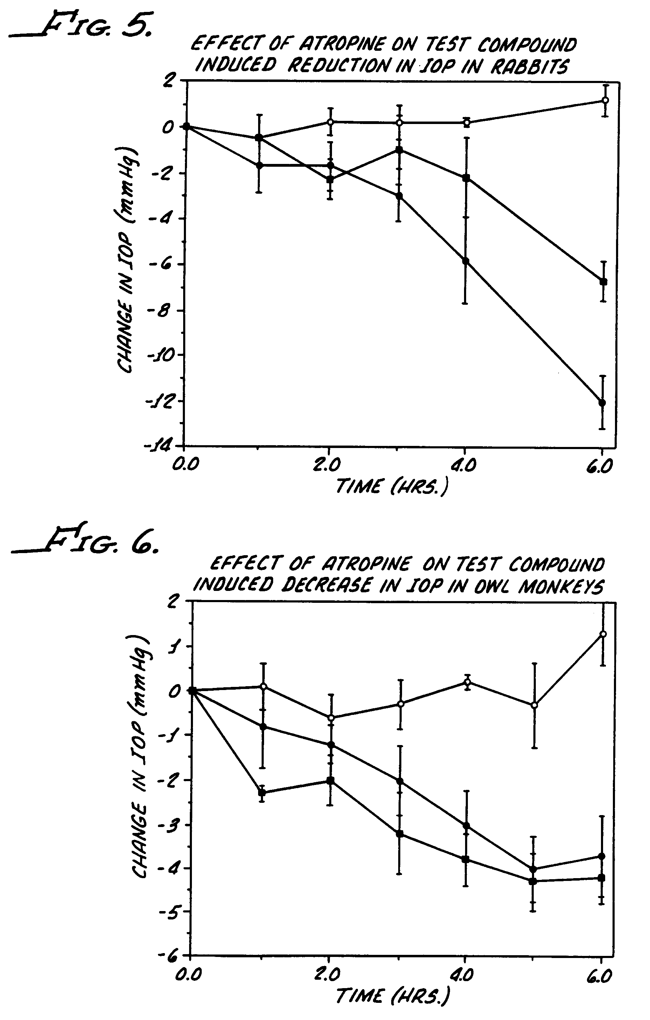

An observation of such an effect for a muscarinic agonist is the first of its kind, especially in normotensive rabbits. Thus, the next question was whether the test compound caused reduction in IOP by activating muscarinic receptors. This was investigated by pretreating the animals with atropine, a muscarinic antagonist. In rabbits, coinjection of atropine with the test compound only caused partial inhibition of IOP effect (Figure 5). Topical application of a 0.5% atropine solution every 2 hours had no inhibitory effect either (data not shown). Similarly, topical application of 5 µg/10 µl atropine into eyes of owl monkeys had no inhibitory effect on the IOP reducing effect of the test compound (Figure 6). It is important to note that this concentration of atropine is several orders of magnitude higher than what is required to cause complete inhibition of activity on muscarinic receptors. This suggests that the mechanism of action of the test compound for reducing IOP in both owl monkeys and rabbits may not be entirely via muscarinic receptors.

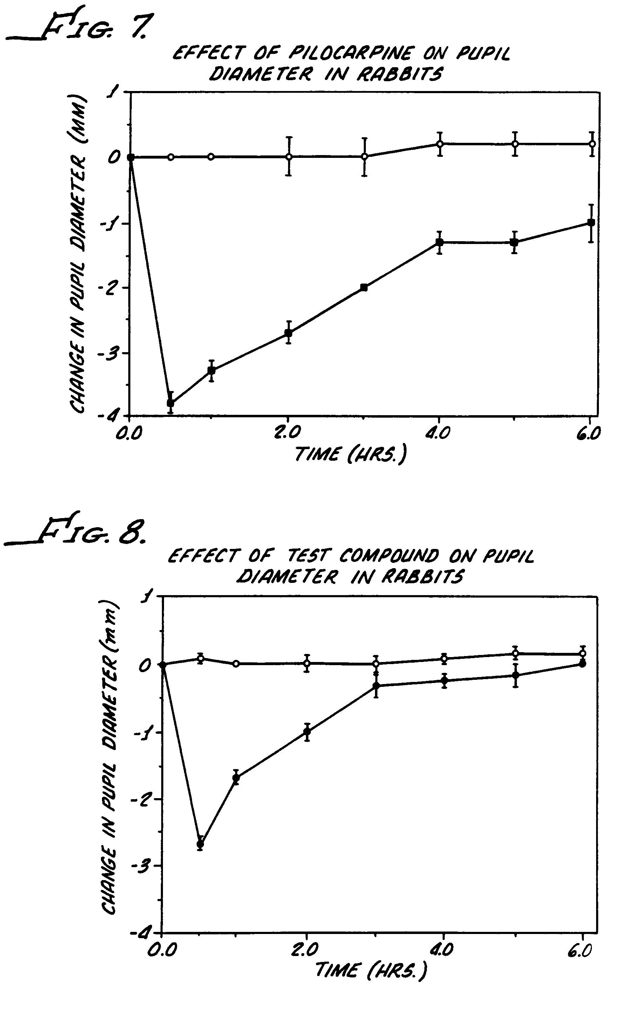

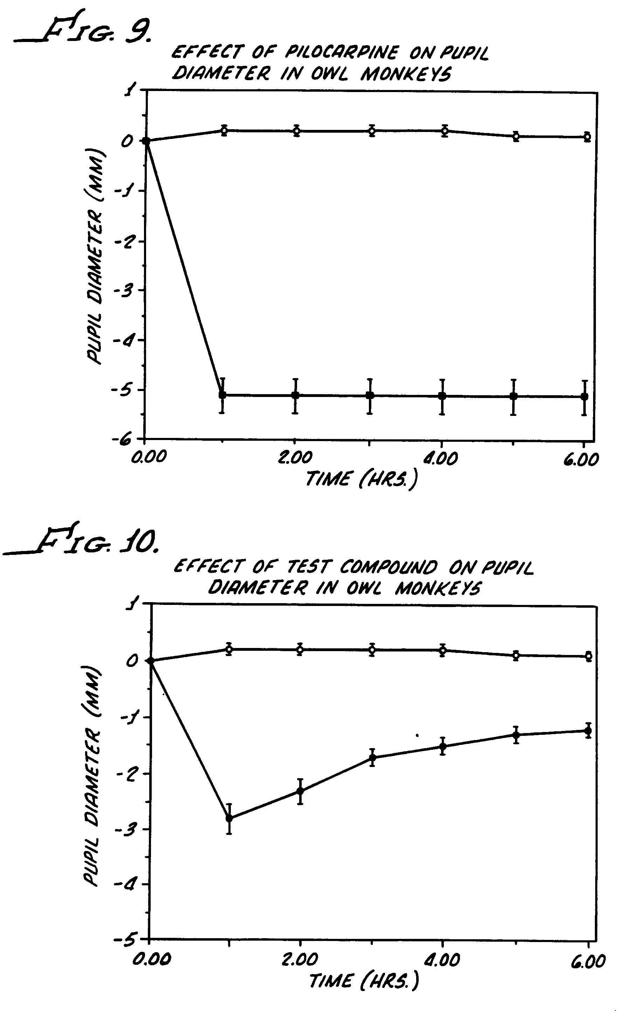

Miosis. Both pilocarpine and the test compound caused miosis in rabbits, but the effect of pilocarpine was greater than that of the test compound (Figures 7 and 8). In both cases, maximum response was obtained at 0.5 hours and it was followed by recovery through the rest of the experimental period.

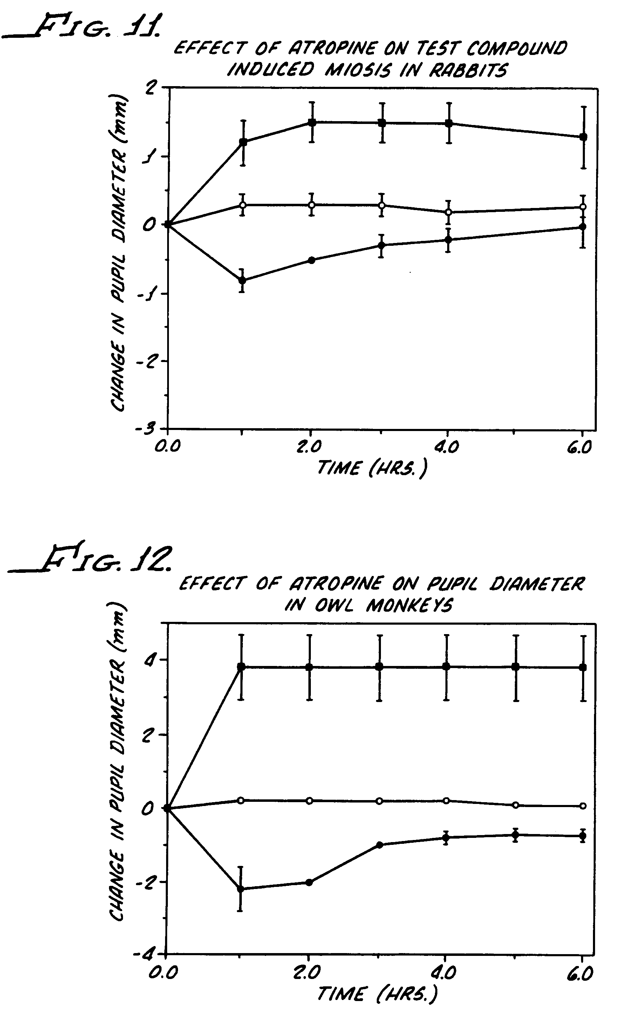

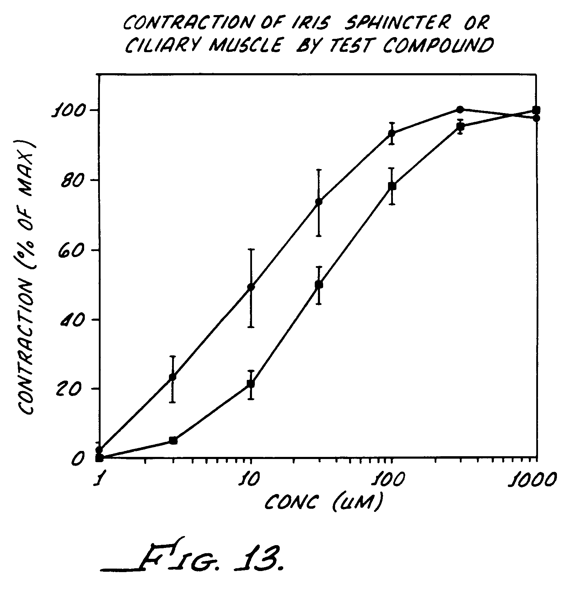

The miotic effect of pilocarpine was severe in owl monkeys, causing complete closure of the pupil for longer than 24 hours (Figure 9). Miosis caused by the test compound, on the other hand, was much less with a decrease of 48% at 1 hour which recovered to 15% of 0 time within 3 to 6 hours (Figure 10). The miotic effect of the test compound in both species was completely inhibited by atropine (Figures 11 and 12). This indicates the involvement of muscarinic receptors of the iris sphincter muscle.

### Example 2

### In vitro studies.

### Methods

Iris sphincter and ciliary smooth muscles were excised from freshly enucleated cat eyes. The tissues were placed in an organ bath containing Tyrode's solution (composition in mM: NaCl 137, KCl 2.7, CaCl₂ 1.8, MgCl₂ 1.05, NaHCO₃ 11.9, NaH₂PO₄ 0.42, D-glucose 5.6) at 37°C, aerated with 95% oxygen and 5% carbon dioxide. One end of the tissue was fastened to a hook in the bath and the other end was attached to a force displacement transducer connected to a polygraph. The tissues were allowed to equilibrate for 60 minutes under a load of 100-200 mg tension. After equilibration, suboptimal concentrations of bethanechol (10-30 µM) were used repeatedly to prime the tissues. Increasing concentrations of the test compound (0.1 - 1000 µM) were then added into the bath to generate cumulative dose response. To test the effect of muscarinic antagonist, the tissues were incubated with 10 nM atropine for 30 minutes before treatment with the test compound.

Agonist activity was expressed as ED₅₀ - a concentration that caused 50% of maximum contraction in a cumulative dose response curve. Results were from three or more experiments expressed as mean ± S.E.

### Results and Discussion

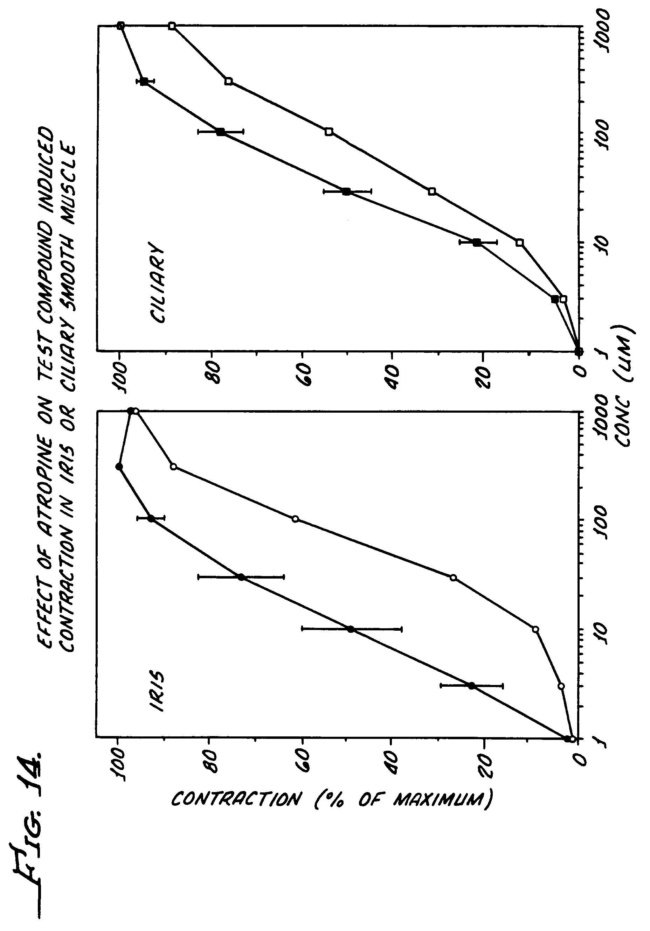

This study was done to quantitatively evaluate the activity of pyridine-1,2,3,6-tetrahydro-5-(5-isoxazolyl)-1-methyl-ethanedioate ("test compound") on the ciliary and iris sphincter smooth muscles in order to understand the mechanism of action of the compound. Previous studies have shown that muscarinic agonists cause a decrease in IOP by contracting the ciliary muscle [Kaufman, P.L. and Barany, E.H., Invest. Ophthalmol. 15, 793-807 (1976)]. Its action on the iris sphincter muscle causes miosis which is an undesired side effect. In this study, the test compound caused a concentration related contraction of both ciliary and iris sphincter muscles with ED₅₀ of 30 and 20 µM, respectively. This compound was less potent than pilocarpine which had ED₅₀ of 4 µM in both tissues (Figure 13). Atropine caused competitive inhibition of contraction indicating that this compound activates muscarinic receptors in these tissues (Figures 14 and 15). However, the effect of atropine was 2.5-times greater in inhibiting the response of the iris than that of the ciliary. This appears to agree with the data from the in vivo studies presented in Example 1, whereby miosis was completely and IOP partially affected by atropine.

The data presented in Examples 1 and 2 clearly indicate that the test compound caused decrease in IOP in two animal models, i.e. rabbits and owl monkeys. Although such an effect has been previously demonstrated for muscarinic agents in owl monkeys [Lamble, J.W. and Lamble, A.P., Invest. Ophthalmol. 15, 848-851 (1976)], this is the first time that such an observation was made in rabbits. Screening of compounds for use in glaucoma therapy is mostly done using rabbits. Thus the fact that the test compound and its analogs lower IOP in rabbits is an added advantage over pilocarpine. The test compound was also more effective in lowering IOP in owl monkeys although the in vitro studies have shown that it was less potent than pilocarpine. From the in vitro studies it was also clear that these compounds can cause contraction of the ciliary muscle of the cat by an atropine sensitive receptor. However, the lack of complete inhibition of its IOP lowering effect by very high concentrations of atropine suggests that the test compounds may have another mechanism of action of reducing IOP. A non-muscarinic activity of such compounds has not been previously observed.

The ability of the compounds disclosed in the present invention to decrease IOP with long duration and the reduced and short-lived miosis accompanying the IOP reducing activity make these compounds excellent candidates for glaucoma therapy.

## Claims

1. The use of a compound of the formula (I) 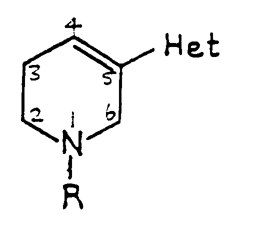 wherein R is hydrogen or an acyclic hydrocarbon group of 1 to about 6 carbon atoms, and Het is a five membered heterocyclic ring containing two hetero-atoms selected from N, O and S, unsubstituted or substituted with an acyclic hydrocarbon group of up to about 10 carbon atoms, an alicyclic hydrocarbon group of about 3 to about 6 carbon atoms, or with an -OR₁ group in which R₁ is an acyclic hydrocarbon group of 1 to about 6 carbon atoms, or a pharmaceutically acceptable acid addition salt thereof, for the preparation of a medicament for the treatment of ocular hypertension.

2. The use according to Claim 1 wherein Het is a five membered heterocyclic ring containing oxygen and nitrogen, or sulfur and nitrogen as heteroatom.

3. The use according to Claim 2 wherein Het is selected from the group consisting of isoxazole and thiazole ring.

4. The use according to Claim 3 wherein R is an alkyl group having from 1 to 4 carbon atoms.

5. The use according to Claim 4 wherein R is methyl.

6. The use according to Claim 5 wherein the Het heterocyclic ring is unsubstituted or substituted with an alkyl group having from 1 to about 8 carbon atoms.

7. The use according to Claim 5 wherein said heterocyclic ring is substituted with an alkoxy group having from 1 to about 6 carbon atoms in the alkyl moiety.

8. The use according to Claim 7 wherein said alkoxy group has from 1 to 4 carbon atoms in the alkyl moiety.

9. The use according to Claim 3 wherein R is hydrogen.

10. The use of Claim 9 wherein the Het heterocyclic ring is substituted with an alkyl group having from 1 to about 4 carbon atoms.

11. The use according to Claim 1 wherein said compound of formula (I) is selected from the group consisting of
pyridine-1,2,3,6-tetrahydro-5-(5-isoxazolyl)-1-methyl-ethanedioate and
pyridine-1,2,3,6-tetrahydro-5-(2-methyl-4-thiazolyl)-1-methyl-ethanedioate;
and a pharmaceutically acceptable acid addition salt of either of these compounds.

12. The use according to Claim 11 wherein said compound is pyridine-1,2,3,6-tetrahydro-5-(5-isoxazolyl)-1-methyl-ethanedioate, or a pharmaceutically acceptable acid addition salt thereof.

13. An ophthalmic solution for the treatment of ocular hypertension, comprising an amount sufficient to treat ocular hypertension of a compound of formula (I) as defined in Claim 1, or a pharmaceutically acceptable acid addition salt thereof, in admixture with a non-toxic, ophthalmically acceptable liquid vehicle, packaged in a container suitable for metered application.

14. The ophthalmic solution according to Claim 13 wherein said compound of formula (I) is selected from the group consisting of
pyridine-1,2,3,6-tetrahydro-5-(5-isoxazolyl)-1-methyl-ethanedioate and
pyridine-1,2,3,6-tetrahydro-5-(2-methyl-4-thiazolyl)-1- methyl-ethanedioate;
and a pharmaceutically acceptable acid addition salt of either of these compounds.

15. The ophthalmic solution according to Claim 14, wherein said compound is pyridine-1,2,3,6-tetrahydro-5-(5-isoxazolyl)-1-methyl-ethanedioate or a pharmaceutically acceptable acid addition salt thereof.

16. A pharmaceutical product, comprising
a container adapted to dispense its contents in metered form; and
an ophthalmic solution therein, as defined in Claim 13.

17. The pharmaceutical product according to Claim 16 comprising an ophthalmic solution wherein said compound of formula (I) is selected from the group consisting of
pyridine-1,2,3,6-tetrahydro-5-(5-isoxazolyl)-1-methyl-ethanedioate and
pyridine-1,2,3,6-tetrahydro-5-(2-methyl-4-thiazolyl)-1-methyl-ethanedioate;
and a pharmaceutically acceptable acid addition salt of either of these compounds.

18. The pharmaceutical product according to Claim 17 wherein said compound is pyridine-1,2,3,6-tetrahydro-5-(5-isoxazolyl)-1-methyl-ethanedioate or a pharmaceutically acceptable acid addition salt thereof.

## Patentansprüche

1. Verwendung einer Verbindung der Formel (I) 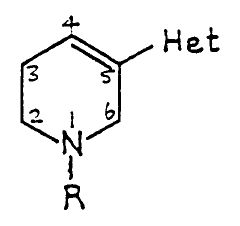 worin R Wasserstoff oder eine acyclische Kohlenwasserstoffgruppe mit 1 bis etwa 6 Kohlenstoffatomen ist und Het einen 5-gliedrigen heterocyclischen Ring, der zwei unter N, O und S ausgewählte Heteroatome enthält und der unsubstituiert ist oder mit einer acyclischen Kohlenwasserstoffgruppe mit bis etwa 10 Kohlenstoffatomen, einer alicyclischen Kohlenwasserstoffgruppe mit etwa 3 bis etwa 6 Kohlenstoffatomen oder mit einer -OR₁-Gruppe substituiert ist, in der R₁ eine acyclische Kohlenwasserstoffgruppe mit 1 bis etwa 6 Kohlenstoffatomen ist, darstellt,
oder eines pharmazeutisch geeigneten Säureadditionssalzes dieser Verbindung zur Herstellung eines Arzneimittels zur Behandlung von erhöhtem Augendruck.

2. Verwendung nach Anspruch 1, wobei Het einen 5-gliedrigen heterocyclischen Ring, der Sauerstoff und Stickstoff oder Schwefel und Stickstoff als Heteroatome enthält, bedeutet.

3. Verwendung nach Anspruch 2, wobei Het aus der aus Isoxazol- und Thiazol-Ringen bestehenden Gruppe ausgewählt ist.

4. Verwendung nach Anspruch 3, wobei R eine Alkylgruppe mit 1 bis 4 Kohlenstoffatomen bedeutet.

5. Verwendung nach Anspruch 4, wobei R Methyl ist.

6. Verwendung nach Anspruch 5, wobei der heterocyclische Ring Het unsubstituiert ist oder mit einer Alkylgruppe mit 1 bis etwa 8 Kohlenstoffatomen substituiert ist.

7. Verwendung nach Anspruch 5, wobei der heterocyclische Ring mit einer Alkoxygruppe substituiert ist, die 1 bis etwa 6 Kohlenstoffatome in dem Alkylrest enthält.

8. Verwendung nach Anspruch 7, wobei die Alkoxygruppe 1 bis 4 Kohlenstoffatome in dem Alkylrest enthält.

9. Verwendung nach Anspruch 3, wobei R Wasserstoff ist.

10. Verwendung nach Anspruch 9, wobei der heterocyclische Ring Het mit einer Alkylgruppe mit 1 bis etwa 4 Kohlenstoffatomen substituiert ist.

11. Verwendung nach Anspruch 1, wobei die Verbindung der Formel (I) aus der Gruppe ausgewählt ist, die aus
Pyridin-1,2,3,6-tetrahydro-5-(5-isoxazolyl)-1-methyl-ethandioat und
Pyridin-1,2,3,6-tetrahydro-5-(2-methyl-4-thiazolyl)-1-methyl-ethandioat
und einem pharmazeutisch geeigneten Säureadditionssalz jeder dieser Verbindungen besteht.

12. Verwendung nach Anspruch 11, wobei die Verbindung Pyridin-1,2,3,6-tetrahydro-5-(5-isoxazolyl)-1-methyl-ethandioat oder ein pharmazeutisch geeignetes Säureadditionssalz davon ist.

13. Ophthalmische Lösung zur Behandlung von erhöhtem Augendruck, enthaltend eine zur Behandlung von erhöhtem Augendruck ausreichende Menge einer Verbindung der Formel (I) gemäß der Definition in Anspruch 1 oder eines pharmazeutisch geeigneten Säureadditionssalzes dieser im Gemisch mit einem nicht-toxischen für die Augenheilkunde geeigneten flüssigen Trägermaterial, die in einem zur dosierten Anwendung geeigneten Gefäß verpackt ist.

14. Ophthalmische Lösung nach Anspruch 13, wobei die Verbindung der Formel (I) aus der Gruppe ausgewählt ist, die aus
Pyridin-1,2,3,6-tetrahydro-5-(5-isoxazolyl)-1-methyl-ethandioat und
Pyridin-1,2,3,6-tetrahydro-5-(2-methyl-4-thiazolyl)-1-methyl-ethandioat
und einem pharmazeutisch geeigneten Säureadditionssalz jeder dieser Verbindungen besteht.

15. Ophthalmische Lösung nach Anspruch 14, wobei die Verbindung Pyridin-1,2,3,6-tetrahydro-5-(5-isoxazolyl)-1-methyl-ethandioat oder ein pharmazeutisch geeignetes Säureadditionssalz dieser ist.

16. Arzneimittel, welches ein Gefäß, das zur Abgabe seines Inhalts in dosierter Form ausgebildet ist, und eine darin befindliche ophthalmische Lösung gemäß der Definition in Anspruch 13 umfaßt.

17. Arzneimittel nach Anspruch 16, das eine ophthalmische Lösung umfaßt, wobei die Verbindung der Formel (I) aus der Gruppe ausgewählt ist, die aus
Pyridin-1,2,3,6-tetrahydro-5-(5-isoxazolyl)-1-methyl-ethandioat und
Pyridin-1,2,3,6-tetrahydro-5-(2-methyl-4-thiazolyl)-1-methyl-ethandioat
und einem pharmazeutisch geeigneten Säureadditionssalz jeder dieser Verbindungen besteht.

18. Arzneimittel nach Anspruch 17, wobei die Verbindung Pyridin-1,2,3,6-tetrahydro-5-(5-isoxazolyl)-1-methyl-ethandioat oder ein pharmazeutisch geeignetes Säureadditionssalz dieser ist.

## Revendications

1. Utilisation d'un composé répondant à la formule (I) : 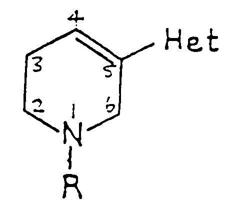 dans laquelle R représente l'atome d'hydrogène ou un groupe hydrocarboné acyclique de 1 à environ 6 atomes de carbone, et Het représente un noyau hétérocyclique de cinq membres contenant deux hétéroatomes choisis parmi N, O et S, non substitué ou substitué par un groupe hydrocarboné acyclique comportant jusqu'à 10 atomes de carbone environ, par un groupe hydrocarboné alicyclique d'environ 3 à environ 6 atomes de carbone, ou par un groupe -OR₁ où R₁ représente un groupe hydrocarboné acyclique de 1 à environ 6 atomes de carbone, ou l'un de ses sels d'addition d'acide acceptable au plan pharmaceutique, pour la préparation d'un médicament destiné au traitement de l'hypertension oculaire.

2. Utilisation selon la revendication 1, dans laquelle Het représente un noyau hétérocyclique de cinq membres contenant un atome d'oxygène et un atome d'azote, ou un atome de soufre et un atome d'azote en tant qu'hétéroatomes.

3. Utilisation selon la revendication 2, dans laquelle Het est choisi parmi le groupe consistant en des noyaux isoxazole et thiazole.

4. Utilisation selon la revendication 3, dans laquelle R représente un groupe alcoyle de 1 à 4 atomes de carbone.

5. Utilisation selon la revendication 4, dans laquelle R représente le groupe méthyle.

6. Utilisation selon la revendication 5, dans laquelle le noyau hétérocyclique Het est non substitué ou substitué par un groupe alcoyle de 1 à environ 8 atomes de carbone.

7. Utilisation selon la revendication 5, dans laquelle ledit noyau hétérocyclique est substitué par un groupe alkoxy dont le fragment alcoyle comporte de 1 à environ 6 atomes de carbone.

8. Utilisation selon la revendication 7, dans laquelle ledit groupe alkoxy comporte de 1 à 4 atomes de carbone dans le fragment alcoyle.

9. Utilisation selon la revendication 3, dans laquelle R représente l'atome d'hydrogène.

10. Utilisation selon la revendication 9, dans laquelle ledit noyau hétérocyclique Het est substitué par un groupe alcoyle de 1 à environ 4 atomes de carbone.

11. Utilisation selon la revendication 1, dans laquelle le composé répondant à la formule (I) est choisi parmi le groupe consistant en
pyridine-1,2,3,6-tétrahydro-5-(5-isoxazolyl)-1-méthyl-éthanedioate et
pyridine-1,2,3,6-tétrahydro-5-(2-méthyl-4-thiazolyl)-1-méthyl-éthanedioate ;
et un sel d'addition d'acide acceptable au plan pharmaceutique de l'un ou l'autre de ces deux composés.

12. Utilisation selon la revendication 11, dans laquelle le composé est le
pyridine-1,2,3,6-tétrahydro-5-(5-isoxazolyl)-1-méthyl-éthanedioate, ou l'un de ses sels d'addition d'acide acceptable au plan pharmaceutique.

13. Solution ophtalmique destinée au traitement de l'hypertension oculaire, comprenant une quantité suffisante pour traiter l'hypertension oculaire d'un composé répondant à la formule (I) tel que défini dans la revendication 1, ou de l'un de ses sels d'addition d'acide acceptable au plan pharmaceutique, en mélange avec un véhicule liquide non toxique acceptable au plan ophtalmique, conditionnée dans un récipient approprié pour une application dosée.

14. Solution ophtalmique selon la revendication 13, dans laquelle ledit composé répondant à la formule (I) est choisi parmi le groupe consistant en
pyridine-1,2,3,6-tétrahydro-5-(5-isoxazolyl)-1-méthyl-éthanedioate et
pyridine-1,2,3,6-tétrahydro-5-(2-méthyl-4-thiazolyl)-1-méthyl-éthanedioate ;
et un sel d'addition d'acide acceptable au plan pharmaceutique de l'un ou l'autre de ces deux composés.

15. Solution ophtalmique selon la revendication 14, dans laquelle le composé est le pyridine-1,2,3,6-tétrahydro-5-(5-isoxazolyl)-1-méthyl-éthanedioate, ou l'un de ses sels d'addition d'acide acceptable au plan pharmaceutique.

16. Produit pharmaceutique, comprenant
un récipient adapté pour délivrer son contenu sous une forme dosée ; et
une solution ophtalmique disposée dans ledit récipient, telle que définie dans la revendication 13.

17. Produit pharmaceutique selon la revendication 16 comprenant une solution ophtalmique dans laquelle ledit composé répondant à la formule (I) est choisi parmi le groupe consistant en
pyridine-1,2,3,6-tétrahydro-5-(5-isoxazolyl)-1-méthyl-éthanedioate et
pyridine-1,2,3,6-tétrahydro-5-(2-méthyl-4-thiazolyl)-1-méthyl-éthanedioate ;
et un sel d'addition d'acide acceptable au plan pharmaceutique de l'un ou l'autre de ces deux composés.

18. Produit pharmaceutique selon la revendication 17, dans lequel ledit composé est le pyridine-1,2,3,6-tétrahydro-5-(5-isoxazolyl)-1-méthyl-éthanedioate, ou l'un de ses sels d'addition d'acide acceptable au plan pharmaceutique.
